(19) Europäisches Patentamt
European Patent Office
Office européen des brevets

(11) EP 1 407 758 B1

(12) EUROPÄISCHE PATENTSCHRIFT

(45) Veröffentlichungstag und Bekanntmachung des Hinweises auf die Patenterteilung:
**09.07.2008 Patentblatt 2008/28**

(51) Int Cl.:
***A61K 8/29*** *(2006.01)* ***A61K 8/49*** *(2006.01)*
***A61Q 17/04*** *(2006.01)*

(21) Anmeldenummer: **03019964.0**

(22) Anmeldetag: **03.09.2003**

(54) **Fettfreie Sonnenschutzmittel**

Fat-free sun compositions

Compositions solaires non grasses

(84) Benannte Vertragsstaaten:
**AT BE BG CH CY CZ DE DK EE ES FI FR GB GR HU IE IT LI LU MC NL PT RO SE SI SK TR**

(30) Priorität: **10.10.2002 DE 10247357**

(43) Veröffentlichungstag der Anmeldung:
**14.04.2004 Patentblatt 2004/16**

(73) Patentinhaber: **Beiersdorf AG**
**20245 Hamburg (DE)**

(72) Erfinder:
- **Müller, Anja**
**23843 Rümpel (DE)**
- **Grotelüschen, Birgit**
**22459 Hamburg (DE)**
- **Gronau, Annette**
**22763 Hamburg (DE)**

(56) Entgegenhaltungen:
**EP-A- 1 371 359**
**WO-A-03/032939**
**WO-A-03/039507**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 1 407 758 B1

## Beschreibung

Die vorliegende Erfindung betrifft kosmetische und/oder dermatologische Zubereitungen enthaltend Titandioxid in Pigmentform, 2-Phenylbenzimidazol-5-sulfonsäure und/oder deren Salze sowie 2,2'-Methylen-bisr(6-(2H-benzotriazol-2-yl)-4-(1, 1,3,3-tetramethylbutyl)-phenol) neben gegebenenfalls anderen kosmetischen und/oder dermatologischen Wirk-, Hilfs- und Zusatzstoffen, das Verfahren zur Herstellung sowie die Verwendung derartiger Zubereitungen.

Der Trend weg von der vornehmen Blässe hin zur "gesunden, sportlich braunen Haut" ist seit Jahren ungebrochen. Um diese zu erzielen setzen die Menschen ihre Haut der Sonnenstrahlung aus, da diese eine Pigmentbildung im Sinne einer Melaninbildung hervorruft. Die ultraviolette Strahlung des Sonnenlichtes hat jedoch auch eine schädigende Wirkung auf die Haut. Neben der akuten Schädigung (Sonnenbrand) treten Langzeitschäden wie ein erhöhtes Risiko an Hautkrebs zu erkranken bei übermäßiger Bestrahlung mit Licht aus dem UVB-Bereich (Wellenlänge: 280-320 nm) auf. Die übermäßige Einwirkung der UVB- und UVA-Strahlung (Wellenlänge: 320-400 nm) führt darüber hinaus zu einer Schwächung der elastischen und kollagenen Fasern des Bindegewebes. Dies führt zu zahlreichen phototoxischen und photoallergischen Reaktionen und hat eine vorzeitige Hautalterung zur Folge.

Zum Schutz der Haut wurden daher eine Reihe von Lichtschutzfiltersubstanzen entwickelt, die in kosmetischen Zubereitungen eingesetzt werden können. Diese UVA- und UVB-Filter sind in den meisten Industrieländern in Form von Positivlisten wie dem Anlage 7 der Kosmetikverordnung zusammengefasst.

Um die Wirksamkeit der Lichtschutzfilter für die Haut abschätzen zu können, wurde in den 50er Jahren der Lichtschutzfaktor (LSF oder LF) bzw. Sonnenschutzfaktor (SF, engl. sun protection factor SPF) von Schulze eingeführt. Er definiert sich wie folgt:

$$LSF = \frac{MED\ geschützte\ Haut}{MED\ ungeschützte\ Haut}$$

*MED* = Erythemschwellendosis (engl. minimum erythemal dose)

Als UV-Lichtschutzfilter können sowohl anorganische Pigmente wie zum Beispiel Titandioxid als auch organische Verbindungen, meist aromatische Substanzen mit ausgeprägtem $\pi$-Elektronensystem, eingesetzt werden.

Eine außerordentlich bedeutsame Klasse an organischen UV-Lichtschutzfiltern leitet sich von den Benzimidazolsulfonsäuren ab.

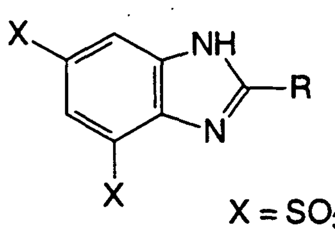

X = $SO_3H$ oder H

Grundgerüst der Benzimidazolsulfonsäurederivate

Eine weitere außerordentlich wichtige Klasse organischer Lichtschutzfilter baut auf dem Strukturelement des Benzotriazols auf.

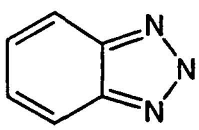

Grundgerüst der Benztriazolderivate

Sonnenschutzmittel gibt es in verschiedenen Ausführungsformen. Besonders beliebt sind wasserfeste Produkte, bei denen die Lichtschutzfilter bei Wasserkontakt nicht sofort abgewaschen werden. Eigentlich müsste man bei diesen Produkten von wasserabweisenden oder wasserbeständigen Sonnenschutzmitteln sprechen, da die Wirkung dieser Mittel im Laufe der Zeit durch Wasserkontakt verloren geht, doch hat sich bei den Verbrauchern der etwas irreführende Begriff wasserfest durchgesetzt. Die Herstellung solcher Produkte wird ermöglicht durch

- Verwendung hydrophober Anwendungsformen z.B. Ölen, Emulsionen, Lipogelen, Pasten auf Fett/Wachsbasis
- Einarbeitung hydrophober Zusätze z.B. Silikonderivate, alkyliertes Polyvinylpyrrolidon.

Der Nachteil dieser Produkte besteht in dem hohen Fettanteil der Zubereitungen, welcher nötig ist, um Wasser abzuweisen und feste lipophile UV-Lichtschutzfilter in Lösung zu bringen. Diese Produkte fühlen sich immer sehr fettig an und führen dazu, dass Sand großflächig auf der Haut haften bleibt.

An sich ist die Verwendung von Fettkomponenten in kosmetischen oder dermatologischen Zubereitungen unbedenklich. Dennoch können auch Fettkomponenten wie letztlich jede chemische Substanz, im Einzelfalle allergische oder auf Überempfindlichkeit des Anwenders beruhende Reaktionen hervorrufen. So sind beispielsweise auch verschiedene Öle in Verdacht, bei Exposition von Sonnenlicht Lichtdermatosen auszulösen, welche auch als "Mallorca-Akne" bezeichnet werden.

Fetthaltige kosmetische und dermatologische Zubereitungen haben darüber hinaus den Nachteil, daß sie comedogen wirken können, d. h. sie können die Bildung von Hauterscheinungen hervorrufen oder unterstützen, welche sich durch nicht entzündliche und entzündliche Knötchen auszeichnen. Ausgehend von verstopften Haarfollikeln (Komedonen) können solche Hauterscheinungen zu Pustel-, Abszess- und Narbenbildung führen. Am häufigsten ist die Acne vulgaris, die vorwiegend in der Pubertät auftritt. Ursächliche Bedingungen sind die Verhornung und Verstopfung der Haarfollikel-Mündung.

Es war daher die Aufgabe der vorliegenden Erfindung, den Mängeln des Standes der Technik abzuhelfen und wasserfeste Sonnenschutzformulierungen zu entwickeln die als weniger fettig empfunden werden.

Vollkommen überraschend und für den Fachmann nicht vorhersehbar wird die Aufgabe gelöst durch eine fettfreie kosmetische und/oder dermatologische Zubereitung enthaltend

a) Titandioxid in Pigmentform,
b) 2-Phenylbenzimidazol-5-sulfonsäure und/oder deren Salze sowie
c) 2,2'-Methylen-bis-(6-(2H-benzotriazol-2-yl)-4-(1,1,3,3-tetramethylbutyl)-phenol) neben gegebenenfalls anderen kosmetischen und/oder dermatologischen Wirk-, Hilfs- und Zusatzstoffen.

Die erfindungsgemäßen kosmetischen und/oder dermatologischen Zubereitungen können fettfrei hergestellt werden, sind sandabweisend und im herkömmlichen Sinne wasserfest. Sie lassen sich erfindungsgemäß als Sonnenschutzmittel verwenden.

Im Rahmen dieser Schrift werden Öle, Fette und Wachse kollektiv als "Fette" bzw. "Fettkomponenten" bezeichnet. Dementsprechend sind unter den Begriffen "Fette" und "Fettkomponenten" im Sinne der vorliegenden Erfindung die folgenden Verbindungen zu verstehen:

- Kohlenwasserstoffe
- Triglyceride
- Fettester bzw. Esteröle

In der Gruppe der Kohlenwasserstoffe werden die verschiedenen Fraktionen der mineralischen Öle und Fette sowie Squalen und Squalan zusammengefasst. Den Verbindungen dieser Gruppe ist gemeinsam, daß sie aus geradkettigen oder verzweigtkettigen Kohlenwasserstoffen aufgebaut sind. Zu den Kohlenwasserstoffen gehören z. B. Paraffinöl, Vaseline, Hartparaffin, mikrokristallines Wachs, Mineralöle, Ozokerit und Ceresin.

Triglyceride sind vollständige Ester des Glycerins mit Fettsäuren. Die Verbindungen dieser Gruppe bilden üblicherweise einen wesentlichen Bestandteil von lipidhaltigen kosmetischen Präparaten. In diese Gruppe fallen die natürlich vorkommenden (pflanzlichen und tierischen) Öle (beispielsweise Avocado-, Oliven-, Maiskeim-, Nerz-, Ricinus-, Soja-, Sonnenblumen- und Sesamöl, um nur einige zu nennen) und Fette (z. B. Japanwachs, Kakaobutter und dergleichen mehr). Ferner gehören auch synthetische Triglyceride, welche durch Veresterung von Fettsäuren mit Glycerin hergestellt werden, zu dieser Gruppe.

Durch Veresterung von Fettalkoholen mit organischen Mono-, Di- und Polycarbonsäuren oder kurzkettigen Alkoholen mit langkettigen Fettsäuren lässt sich eine große Zahl von Fettestern herstellen, die üblicherweise in kosmetischen Präparaten sehr breit eingesetzt werden. In diese Gruppe fallen u. a. Monocarbonsäureester (beispielsweise Butylstearat, Cetylpalmitat, Decyloleat, 2-Ethylhexylpalmitat, Hexyllaurat, Isopropylisostearat, -lanolat,- laurat, -linoleat,

-palmitat, -stearat, um nur einige zu nennen) Dicarbonsäureester, (z. B. Adipinsäurediisopropylester, Cetyl-, Lauryl- und Myristyllactat und Diglycerinester der Capryl-, Caprin- und Bernsteinsäure und dergleichen) sowie Alkylbenzoate.

Zwar beschreibt der Stand der Technik in den Schriften EP 1 371 359, WO 03/039507 und WO 03/032939 Zubereitungen, welche die erfindungsgemäße UV-Filterkombination enthalten, doch konnten diese Schriften nicht den Weg zur vorliegenden Erfindung weisen, die die beschriebenen Zubereitungen nicht fettfrei im sinne der vorliegenden Offenbarung sind.

Die erfindungsgemäßen kosmetischen und/oder dermatologischen Zubereitungen enthalten Titandioxid in Pigmentform in einer Menge von 0,1 bis 10 Gewichts-% und besonders bevorzugt von 0,5 bis 5 Gewichts-%, bezogen auf das Gesamtgewicht der Formulierung.

Erfindungsgemäß sind dabei sowohl röntgenamorphe als auch nichtröntgenamorphe Titandioxidpigmente.

Die nichtröntgenamorphen Pigmente liegen erfindungsgemäß vorteilhaft in hydrophober Form vor, d.h., dass sie oberflächlich wasserabweisend behandelt sind. Diese Oberflächenbehandlung kann darin bestehen, dass die Pigmente nach an sich bekannten Verfahren mit einer dünnen hydrophoben Schicht versehen werden.

Eines solcher Verfahren besteht beispielsweise darin, dass die hydrophobe Oberflächenschicht nach einer Reaktion gemäß

$$n\ TiO_2 + m\ (RO)_3Si\text{-}R' \text{ -> } n\ TiO_2\ (\text{oberfl.})$$

erzeugt wird. n und m sind dabei nach Belieben einzusetzende stöchiometrische Parameter, R und R' die gewünschten organischen Reste. Beispielsweise in Analogie zu DE-OS 33 14 742 dargestellte hydrophobisierte Pigmente sind von Vorteil.

Vorteilhafte $TiO_2$-Pigmente sind beispielsweise unter den Handelsbezeichnungen T 805 von der Firma Degussa erhältlich.

Die erfindungsgemäßen kosmetischen und/oder dermatologischen Zubereitungen enthalten 2-Phenylbenzimidazol-5-sulfonsäure und/oder deren Salze in einer Menge von 0,1 bis 10 Gewichts-% und besonders bevorzugt in einer Menge von 0,5 bis 5 Gewichts-%, jeweils bezogen auf des Gesamtgewicht der Zubereitung. Hierzu zählen die im Sinne der Erfindung besonders vorteilhafte 2-Phenylbenzimidazol-5-sulfonsäure und ihre Natrium-, Kalium- und Triethanolammoniumsalze [Eusolex 232 (Merck), Neo Heliopan Hydro (H&R), Parsol HS (Givaudan)].

Außerdem enthalten die erfindungsgemäßen kosmetischen und/oder dermatologischen Zubereitungen 2,2'-Methylen-bis-(6-(2H-benzotriazol-2-yl)-4-(1,1,3,3-tetramethylbutyl)-phenol)) [Tinosorb M (Ciba)] in einer Menge von 0,1 bis 10 Gewichts-% und besonders bevorzugt in einer Menge von 0,5 bis 5 Gewichts-%, jeweils bezogen auf das Gesamtgewicht der Zubereitung.

Erfindungsgemäß können die kosmetischen und/oder dermatologischen Lichtschutzformulierungen wie üblich zusammengesetzt sein und dem kosmetischen und/oder dermatologischen Lichtschutz, ferner zur Behandlung, der Pflege und der Reinigung der Haut und/oder der Haare und als Schminkprodukt in der dekorativen Kosmetik dienen.

Zur Anwendung werden die kosmetischen und dermatologischen Zubereitungen erfindungsgemäß in der für Kosmetika üblichen Weise auf die Haut und/oder die Haare in ausreichender Menge aufgebracht.

Die kosmetischen und dermatologischen Zubereitungen können erfindungsgemäß kosmetische Hilfsstoffe enthalten, wie sie üblicherweise in solchen Zubereitungen verwendet werden, z.B. Konservierungsmittel, Bakterizide, Parfüme, Substanzen zum Verhindern des Schäumens, Farbstoffe, weitere Pigmente (z.B. ZnO, $BaSO_4$), die eine färbende Wirkung haben, Verdickungsmittel, anfeuchtende und/oder feuchthaltende Substanzen oder andere übliche Bestandteile einer kosmetischen oder dermatologischen Formulierung wie Alkohole, Polyole, Polymere, Schaumstabilisatoren, Elektrolyte, organische Lösungsmittel oder Silikonderivate.

Ein zusätzlicher Gehalt an Antioxidantien ist im allgemeinen bevorzugt. Erfindungsgemäß können als günstige Antioxidantien alle für kosmetische und/oder dermatologische Anwendungen geeigneten oder gebräuchlichen Antioxidantien verwendet werden.

Vorteilhaft werden die Antioxidantien gewählt aus der Gruppe bestehend aus Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis μmol/kg), ferner (Metall)-Chelatoren (z.B. α-Hydroxyfettsäuren, Palmitinsäure, Phytinsäure, Lactoferrin), α - Hydroxysäuren (z.B. Citronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate, Vitamin A und Derivate sowie Koniferylbenzoat des Benzoëharzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z.B. ZnO, $ZnSO_4$) Selen und dessen Derivate (z.B. Selenmethionin), Stilbene und deren Derivate (z.B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Die Menge der vorgenannten Antioxidantien (eine oder mehrere Verbindungen) in den Zubereitungen beträgt vorzugsweise 0,001 bis 10 Gew.-%, besonders bevorzugt 0,05 - 7 Gew.-%, insbesondere 0,5 - 5 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 - 5 Gew.-%, bezogen auf das Gesamtgewicht der Formulierung, zu wählen.

Sofern Vitamin A, bzw. Vitamin-A-Derivate, bzw. Carotine bzw. deren Derivate das oder die Antioxidantien darstellen, ist vorteilhaft, deren jeweilige Konzentrationen aus dem Bereich von 0,001 - 5 Gew.-%, bezogen auf das Gesamtgewicht der Formulierung, zu wählen.

Darüber hinaus eignen sich ausgewählte erfindungsgemäße Rezepturen, welche z. B. bekannte Antifaltenwirkstoffe wie Flavonglycoside (insbesondere α-Glycosylrutin), Coenzym Q10, Vitamin E und/oder Derivate und dergleichen enthalten, insbesondere vorteilhaft zur Prophylaxe und Behandlung kosmetischer oder dermatologischer Hautveränderungen, wie sie z. B. bei der Hautalterung (z.B. Falten und Fältchen) auftreten. Weiterhin vorteilhaft eignen sie sich gegen das Erscheinungsbild der trockenen bzw. rauhen Haut.

Eine gegebenenfalls vorhandene Lipidphase kann vorteilhaft gebildet werden aus Silikonölen wie Dimethylpolysiloxanen, Diethylpolysiloxanen, Diphenylpolysiloxanen sowie Mischformen daraus. Vorteilhaft ist ein Gehalt an cyclischen oder linearen Silikonölen.

Vorteilhaft wird Cyclomethicon (Octamethylcyclotetrasiloxan) oder Dimethicon als erfindungsgemäß zu verwendendes Silikonöl eingesetzt. Aber auch andere Silikonöle sind vorteilhaft im Sinne der vorliegenden Erfindung zu verwenden, beispielsweise Hexamethylcyclotrisiloxan, Polydimethylsiloxan, Poly(methylphenylsiloxan).

Die wässrige Phase der erfindungsgemäßen Zubereitungen enthält gegebenenfalls vorteilhaft

- Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, ferner Alkohole niedriger C-Zahl, z.B. Ethanol, Isopropanol, 1,2-Propandiol, Glycerin sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z.B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination.

Ferner kann erfindungsgemäß gegebenenfalls von Vorteil sein, die Zubereitungen mit weiteren UVA- und/oder UVB-Filtern zu versehen.

Bestandteil der Erfindung ist außerdem das Verfahren zur Herstellung einer kosmetischen und/oder dermatologischen Zubereitung, dadurch gekennzeichnet dass Titandioxid in Pigmentform und 2,2'-Methylen-bis-(6-(2H-benzotriazol-2-yl)-4-(1,1,3,3-tetramethylbutyl)-phenol) separat oder zusammen in einem oder zwei Teilen der wässrigen Phase der Zubereitung dispergiert werden, 2-Phenylbenzimidazol-5-sulfonsäure und/oder deren Salze in einem weiteren Teil der wässrigen Phase der Zubereitung dispergiert wird und anschließend die wässrigen Phasen vereinigt und homogenisiert werden.

Erfindungsgemäß ist ferner eine kosmetische und/oder dermatologische Zubereitung, die nach dem erfindungsgemäßen Verfahren hergestellt wurde.

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

**1.) Fettfreie, sandabweisende O/W-Emulsionen**

| | 1 | 2 |
|---|---|---|
| Glycerylstearat Citrat | 3.00 | |
| Glycerylstearat SE | | 2.00 |
| Glycerylstearat | | |
| PEG-40 Stearat | | |
| Stearinsäure | | 2.00 |
| Cetearyl Alkohol | | 2.00 |
| Cetyl Alkohol | 3.00 | |
| Stearyl Alkohol | | 0.50 |
| Lanolin Alkohol | | 0.50 |
| Myristyl Alkohol | | 2.00 |
| Phenyl Dibenzimidazol Tetrasulfonsäure | | |
| Phenylbenzmidazol Sulfonsäure | 2,00 | 0,50 |
| Methylen Bis-Benzotriazolyl Tetramethylbutylphenol | 0,50 | 2,00 |
| Terephthaliden Dicamphor Sulfonsäure | | |
| Titandioxid Aluminiumhydroxid/Stearinsäure coating | 3,00 | 2,00 |
| Zinkoxid HP1 | 1,00 | |
| Butylenglycol | | |
| Carbomer | 0.20 | 0.30 |
| Xanthan Gum | 0.40 | 0.60 |
| Dimethicon | | |
| Cyclomethicon | | |
| Tocopheryl Acetat | | |
| Glycerin | 7.50 | 3.50 |
| Farbstoff (wasser- und/oder öllöslich) | 0.25 | |
| DMDM Hydantoin | 0.20 | |
| Propylparaben | | |
| Methylparaben | | |
| NaOH 45% | 0,80 | 0,20 |
| Ethanol | | 1.50 |
| Konkaben LMB ® | | 0.20 |
| Trinatrium EDTA | 1.00 | |
| Phenoxyethanol | 0.30 | 0.50 |
| Parfüm | q.s. | q.s. |
| Aqua | Ad 100 | Ad 100 |

**2.) PIT-Emulsionen (zur Verwendung als Tränkungslösung, Spray oder Aerosol)**

| | 1 | 2 |
|---|---|---|
| Glycerinmonostearat SE | 2,00 | 3,00 |
| Glyceryl Isostearat | | |
| Isoceteth-20 | 0,50 | |
| Ceteareth-12 | 5,00 | |
| Ceteareth-20 | | |
| PEG-100 Stearat | | 1,00 |
| Cetyl Alkohol | 1,00 | |
| Cetyl Palmitat | | |
| Lauryl Methicon Copolyol | | 1,00 |
| Polyglyceryl-2 Dipolyhydroxystearat | | |
| UVASorb® K2A | | |
| Uvinul® A Plus | | |
| Methylen Bis-Benzotriazolyl Tetramethylbutylphenol | 0,50 | 0,50 |
| Phenylbenzmidazol Sulfonsäure | 3,00 | 2,50 |
| Titandioxid | 0,50 | 0,50 |
| Dinatrium Phenyl Dibenzimidazol Tetrasulfonat | 2,00 | |
| Terephthaliden Dicampher Sulfonsäure | | |
| Butyl Methoxydibenzoylmethan | | |
| Drometrizol Trisiloxan | | |
| Ethylhexyl Methoxycinnamat | | |
| Diethylhexyl Butamido Triazon | | |
| Ethylhexyl Triazon | | |
| Octocrylen | | |
| Cyclomethicon | | |
| PVP Hexadecen Copolymer | | |
| Glycerin | 5,00 | |
| Vitamin E Acetat | | |
| 2,6 Diethylhexylnaphthalat | | |
| Iodopropyl Butylcarbamat | | |
| DMDM Hydantoin | | |
| Methylparaben | 0,50 | |
| Phenoxyethanol | 0,40 | |
| Ethylhexyloxyglycerin | 0,30 | |
| Ethanol | | 5,00 |
| Farbstoffe, wasserlöslich | | |
| NaOH 45% | q.s. | q.s. |
| Trisodium EDTA | | 0,14 |
| Parfüm | | 0,20 |

(fortgesetzt)

| | 1 | 2 |
|---|---|---|
| Wasser | ad. 100 | ad. 100 |

**Patentansprüche**

1. Kosmetische und/oder dermatologische Zubereitung enthaltend

   a) Titandioxid in Pigmentform,
   b) 2-Phenylbenzimidazol-5-sulfonsäure und/oder deren Salze sowie
   c) 2,2'-Methylen-bis-(6-(2H-benzotriazol-2-yl)-4-(1,1,3,3-tetramethylbutyl)-phenol) neben gegebenenfalls anderen kosmetischen und/oder dermatologischen Wirk-, Hilfs- und Zusatzstoffen, **dadurch gekennzeichnet, dass** sie frei ist von Ölen, Fetten und Wachsen.

2. Kosmetische und/oder dermatologische Zubereitungen nach Anspruch 1 enthaltend a) Titandioxid in Pigmentform in einer Menge von 0,5 bis 5 Gewichts-%

   b) 2-Phenylbenzimidazol-5-sulfonsäure und/oder deren Salze in einer Menge von 0,5 bis 5 Gewichts-%
   c) 2,2'-Methylen-bis-(6-(2H-benzotriazol-2-yl)-4-(1,1,3,3-tetramethylbutyl)-phenol) in einer Menge von 0,5 bis 5 Gewichts-%, jeweils bezogen auf das Gesamtgewicht der Zubereitung.

3. Verwendung von kosmetischen und/oder dermatologischen Zubereitungen nach einem der Ansprüche 1 oder 2 als fettfreie kosmetische und/oder dermatologische Zubereitung.

4. Verwendung von kosmetischen und/oder dermatologischen Zubereitungen nach einem der Ansprüche 1 oder 2 als sandabweisende kosmetische und/oder dermatologische Zubereitung.

5. Verwendung von kosmetischen und/oder dermatologischen Zubereitungen nach einem der Ansprüche 1 oder 2 als Sonnenschutzmittel.

6. Verwendung von kosmetischen und/oder dermatologischen Zubereitungen nach einem der Ansprüche 1 oder 2 als wasserfestes Sonnenschutzmittel.

7. Verfahren zur Herstellung einer fettfreien kosmetischen und/oder dermatologischen Zubereitung nach einem der vorhergehenden Ansprüche, **dadurch gekennzeichnet dass** Titandioxid in Pigmentform und 2,2'-Methylen-bis-(6-(2H-benzotriazol-2-yl)-4-(1,1,3,3-tetramethylbutyl)-phenol) separat oder zusammen in einem oder zwei Teilen der wässrigen Phase der Zubereitung dispergiert werden, 2-Phenylbenzimidazol-5-sulfonsäure und/oder deren Salze in einem weiteren Teil der wässrigen Phase der Zubereitung dispergiert wird und anschließend die wässrigen Phasen vereinigt und homogenisiert werden.

**Claims**

1. Cosmetic and/or dermatological preparation comprising

   a) titanium dioxide in pigment form,
   b) 2-phenylbenzimidazole-5-sulphonic acid and/or salts thereof and
   c) 2,2'-methylenebis(6-(2H-benzotriazol-2-yl)-4-(1,1,3,3-tetramethylbutyl)phenol) besides optionally other cosmetic and/or dermatological active ingredients, auxiliaries and additives, **characterized in that** it is free from oils, fats and waxes.

2. Cosmetic and/or dermatological preparations according to Claim 1 comprising

   a) titanium dioxide in pigment form in an amount of from 0.5 to 5% by weight
   b) 2-phenylbenzimidazole-5-sulphonic acid and/or salts thereof in an amount of from 0.5 to 5% by weight

c) 2,2'-methylenebis(6-(2H-benzotriazol-2-yl)-4-(1,1,3,3-tetramethylbutyl)phenol) in an amount of from 0.5 to 5% by weight, in each case based on the total weight of the preparation.

**3.** Use of cosmetic and/or dermatological preparations according to one of Claims 1 or 2 as fat-free cosmetic and/or dermatological preparation.

**4.** Use of cosmetic and/or dermatological preparations according to one of Claims 1 or 2 as sand-repellent cosmetic and/or dermatological preparation.

**5.** Use of cosmetic and/or dermatological preparations according to one of Claims 1 or 2 as sun composition.

**6.** Use of cosmetic and/or dermatological preparations according to one of Claims 1 or 2 as water-resistant sun composition.

**7.** Process for the preparation of a fat-free cosmetic and/or dermatological preparation according to one of the preceding claims, **characterized in that** titanium dioxide in pigment form and 2,2'-methylenebis(6-(2H-benzotriazol-2-yl)-4-(1,1,3,3-tetramethylbutyl)phenol) are dispersed separately or together in one or two parts of the aqueous phase of the preparation, 2-phenylbenzimidazole-5-sulphonic acid and/or salts thereof is dispersed in a further part of the aqueous phase of the preparation and then the aqueous phases are combined and homogenized.

**Revendications**

**1.** Préparation cosmétique et/ou dermatologique, contenant

a) du dioxyde de titane sous forme pigmentaire,
b) de l'acide 2-phénylbenzimidazole-5-sulfonique et/ou ses sels ainsi que
c) du 2,2'-méthylènebis-(6-(2H-benzotriazol-2-yl)-4-(1,1,3,3-tétraméthylbutyl)-phénol) en plus, le cas échéant, d'autres substances actives, adjuvants et additifs cosmétiques et/ou dermatologiques, **caractérisée en ce qu'**elle est exempte d'huiles, de graisses et de cires.

**2.** Préparations cosmétiques et/ou dermatologiques selon la revendication 1, contenant

a) du dioxyde de titane sous forme pigmentaire en une quantité de 0,5 à 5% en poids
b) de l'acide 2-phénylbenzimidazole-5-sulfonique et/ou ses sels en une quantité de 0,5 à 5% en poids
c) du 2,2'-méthylènebis-(6-(2H-benzotriazol-2-yl)-4-(1,1,3,3-tétraméthylbutyl)-phénol) en une quantité de 0,5 à 5% en poids, à chaque fois par rapport au poids total de la préparation.

**3.** Utilisation de préparations cosmétiques et/ou dermatologiques selon l'une quelconque des revendications 1 ou 2 comme préparation cosmétique et/ou dermatologique exempte de graisse.

**4.** Utilisation de préparations cosmétiques et/ou dermatologiques selon l'une quelconque des revendications 1 ou 2 comme préparation cosmétique et/ou dermatologique repoussant le sable.

**5.** Utilisation de préparations cosmétiques et/ou dermatologiques selon l'une quelconque des revendications 1 ou 2 comme agent de protection solaire.

**6.** Utilisation de préparations cosmétiques et/ou dermatologiques selon l'une quelconque des revendications 1 ou 2 comme agent de protection solaire résistant à l'eau.

**7.** Procédé pour la préparation d'une préparation cosmétique et/ou dermatologique, exempte de graisse selon l'une quelconque des revendications précédentes, **caractérisé en ce que** le dioxyde de titane sous forme pigmentaire et le 2,2'-méthylènebis-(6-(2H-benzotriazol-2-yl)-4-(1,1,3,3-tétraméthylbutyl)-phénol) sont dispersés, séparément ou ensemble, dans une ou deux parties de la phase aqueuse de la préparation, l'acide 2-phénylbenzimidazole-5-sulfonique et/ou ses sels est/sont dispersé(s) dans une autre partie de la phase aqueuse de la préparation, puis les phases aqueuses sont rassemblées et homogénéisées.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- EP 1371359 A
- WO 03039507 A
- WO 03032939 A
- DE OS3314742 A